(19) Europäisches Patentamt
European Patent Office
Office européen des brevets

(11) Veröffentlichungsnummer: **0 391 169 A2**

# (12) EUROPÄISCHE PATENTANMELDUNG

(21) Anmeldenummer: **90105511.1**

(22) Anmeldetag: **23.03.90**

(51) Int. Cl.5: **C07D 401/04, A61K 31/47, C07D 207/14**

(30) Priorität: **05.04.89 DE 3910920**

(43) Veröffentlichungstag der Anmeldung: **10.10.90 Patentblatt 90/41**

(84) Benannte Vertragsstaaten: **AT BE CH DE DK ES FR GB GR IT LI LU NL SE**

(71) Anmelder: **BAYER AG**

**D-5090 Leverkusen 1 Bayerwerk(DE)**

(72) Erfinder: **Petersen, Uwe, Dr.**
**Auf dem Forst 4**
**D-5090 Leverkusen 1(DE)**
Erfinder: **Schenke, Thomas, Dr.**
**Mühlenstrasse 113**
**D-5060 Bergisch Gladbach 2(DE)**
Erfinder: **Schriewer, Michael, Dr.**
**Am Thelen Siefen 1A**
**D-5068 Odenthal(DE)**
Erfinder: **Krebs, Andreas, Dr.**
**Am Gartenfeld 70**
**D-5068 Odenthal(DE)**
Erfinder: **Grohe, Klaus, Dr.**
**Am Wasserturm 10**
**D-5068 Odenthal(DE)**
Erfinder: **Haller, Ingo, Dr.**
**Dornröschenweg 4**
**D-5600 Wuppertal 1(DE)**
Erfinder: **Metzger, Karl Georg, Dr.**
**Pahlkestrasse 75**
**D-5600 Wuppertal 1(DE)**
Erfinder: **Endermann, Rainer, Dr.**
**In den Birken 152a**
**D-5600 Wuppertal 1(DE)**
Erfinder: **Zeiler, Hans-Joachim, Dr.**
**Elsbeekerstrasse 46**
**D-5620 Velbert(DE)**

(54) **Enantiomerereine 7-(3-Amino-1-pyrrolidinyl)-chinolon-und-naphthyridoncarbonsäuren.**

(57) Die Erfindung betrifft neue enantiomerenreine 7-(3-Amino-1-pyrrolidinyl)-chinolon- und -naphthyridoncarbonsäuren, Verfahren zu ihrer Herstellung sowie diese enthaltende antibakterielle Mittel und Futterzusatzstoffe.

Die Chinolone bzw. Naphthyridone entsprechen der allgemeinen Formel (I)

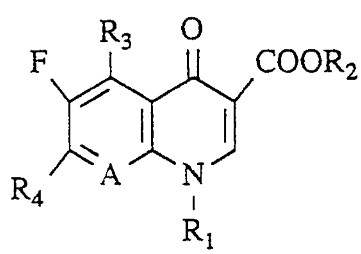

(I)

in der $R_1$, $R_2$, $R_3$, $R_4$ und A die in der Beschreibung angegebene Bedeutung haben.

EP 0 391 169 A2

## Enantiomerenreine 7-(3-Amino-1-pyrrolidinyl)-chinolon-und -naphthyridoncarbonsäuren

Die Erfindung betrifft neue enantiomerenreine 7-(3-Amino-1-pyrrolidinyl)-chinolon- und -naphthyridoncarbonsäuren, Verfahren zu ihrer Herstellung sowie diese enthaltende antibakterielle Mittel und Futterzusatzstoffe.

Es ist bereits eine Reihe von 7-(3-Amino-1-pyrrolidinyl)-chinolon- und -naphthyridoncarbonsäuren bekannt geworden, welche eine hohe antibakterielle Wirkung aufweisen. Als Beispiele seien genannt: 7-(3-Amino-1-pyrrolidinyl)-8-chlor-1-cyclopropyl-6-fluor-1,4-dihydro-4-oxo-3-chinolincarbonsäure (Deutsche Patentanmeldung 3 420 734, Europäische Patentanmeldung 195 316), 7-(3-Amino-1-pyrrolidinyl)-1-cyclopropyl-6,8-difluor-1,4-dihydro-4-oxo-3-chinolincarbonsäure und 7-(3-Amino-1-pyrrolidinyl)-1-cyclopropyl-1,4-dihydro-6-fluor-4-oxo-1,8-naphthyridin-3-carbonsäure (Europäische Patentanmeldung 153 163), 5-Amino-7-(3-amino-1-pyrrolidinyl)-1-cyclopropyl-6,8-difluor-1,4-dihydro-4-oxo-3-chinolin carbonsäure, 7-(3-Amino-1-pyrrolidinyl)-6-fluor-1-(2,4-difluorphenyl)-1,4-dihydro-4-oxo-1,8-naphthyridin-3-carbonsäure (Europäische Patentanmeldung 302 372), 5-Amino-7-(3-amino-1-pyrrolidinyl)-1-ethyl-6,8-difluor-4-oxo-1,4-dihydro-3-chinolincarbonsäure (Europäische Patentanmeldung 172 651).

Es wurde gefunden, daß die neuen enantiomerenreinen Verbindungen der Formel (I)

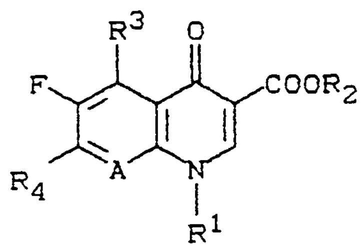

(I) ,

in welcher

$R^1$ für verzweigtes oder geradkettiges Alkyl mit 1 bis 3 Kohlenstoffatomen, Cyclopropyl, Vinyl, 2-Hydroxyethyl, 2-Fluorethyl, gegebenenfalls durch 1 oder 2 Halogenatome substituiertes Phenyl,

$R^2$ für Wasserstoff oder Alkyl mit 1 bis 4 Kohlenstoffatomen,

$R^3$ für Wasserstoff, Fluor, Chlor, Amino, Alkyl mit 1 bis 4 Kohlenstoffatomen,

$R^4$ für einen Rest der Struktur

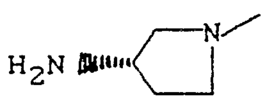

oder

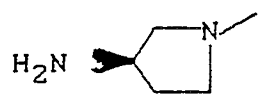

und

A für N oder C-$R^5$ steht, worin $R^5$ für Wasserstoff, Halogen, Methyl, Nitro, Hydroxy oder Methoxy steht oder auch gemeinsam mit $R^1$ eine Brücke der Struktur

$-O-CH_2-CH-CH_3$

bilden kann,

und deren pharmazeutisch verwendbaren Hydrate und Säureadditionssalze sowie die Alkali-, Erdalkali-, Silber-und Guanidiniumsalze der zugrundeliegenden Carbonsäuren gute antibakterielle Eigenschaften aufweisen.

In der Regel sind die S-Formen im MHK-Test antibakteriell wirksamer als die entsprechenden Racemate und eines der Enantiomeren ist verträglicher als das entgegengesetzte Enantiomere.

Das Symbol 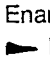 bedeutet eine Bindung oberhalb der Papierebene, während das Symbol 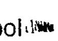 eine Bindung unterhalb der Papierebene darstellt.

Nicht zum Gegenstand der Erfindung gehören 7-[R- und S-(3-Amino)-1-pyrrolidinyl]-6-fluor-1-(2,4-difluorphenyl)-1,4-dihydro-4-oxo-1,8-naphthyridin-3-carbonsäure.

Bevorzugt sind die Verbindungen der Formel (I)

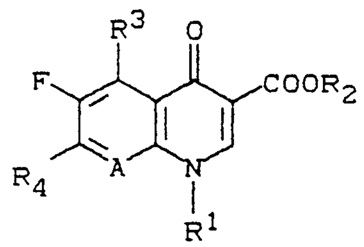

(I) ,

in welcher
$R^1$ für Methyl, Ethyl, Cyclopropyl, 2-Fluorethyl, 4-Fluorphenyl oder 2,4-Difluorphenyl,
$R^2$ für Wasserstoff, Methyl oder Ethyl,
$R^3$ für Wasserstoff, Fluor, Amino oder Methyl,
$R^4$ für einen Rest der Struktur

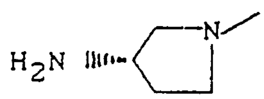 oder 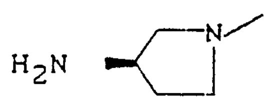 und

A für N oder C-$R^5$ steht, worin $R^5$ für Wasserstoff, Fluor, Chlor, Methyl oder Methoxy steht oder auch gemeinsam mit $R^1$ eine Brücke der Struktur
$-O-CH_2-\underset{|}{C}H-CH_3$
bilden kann,
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I)

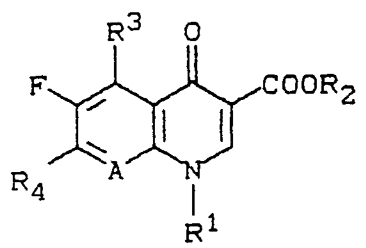

(I)

in welcher
$R^1$ für Methyl, Ethyl, Cyclopropyl, 2-Fluorethyl, 4-Fluorphenyl oder 2,4-Difluorphenyl,
$R^2$ für Wasserstoff, Methyl oder Ethyl,
$R^3$ für Wasserstoff, Fluor, Amino oder Methyl
$R^4$ für einen Rest der Struktur

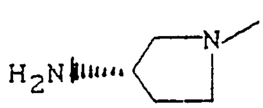 und

A für N oder C-$R^5$ steht, worin $R^5$ für Wasserstoff, Fluor, Chlor, Methyl oder Methoxy steht oder auch gemeinsam mit $R^1$ eine Brücke der Struktur
$-O-CH_2-\underset{|}{C}H-CH_3$
bilden kann,
und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man Verbindungen der Formel (II)

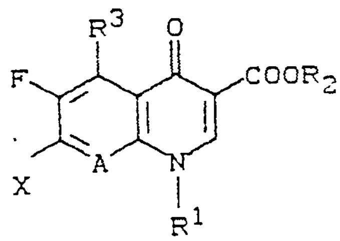

(II) ,

in welcher R', $R^2$, $R^3$ und A die oben angegebene Bedeutung haben und
X für Halogen, insbesondere Fluor oder Chlor steht,
mit Verbindungen der Formel (III) in der R- oder in der S-konfigurierten Form

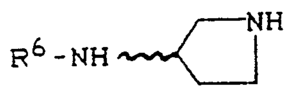

(III) ,

in welcher
$R^6$ für Wasserstoff, Acyl mit 1 bis 3 Kohlenstoffatomen oder Alkoxycarbonyl mit 1 bis 4 Kohlenstoffatomen im Alkoxyteil, insbesondere tert.-Butoxycarbonyl, steht,
gegebenenfalls in Gegenwart von Säurefängern umsetzt und gegebenenfalls vorhandene Schutzgruppen abspaltet.

Verwendet man beispielsweise 6,7,8-Trifluor-1-(2,4-difluor-phenyl)-1,4-dihydro-4-oxo-3-chinolin-carbonsäure und S-(3-tert.-Butoxycarbonylamino)-pyrrolidin als Ausgangsstoffe, so kann der Reaktionsverlauf durch folgendes Formelchema wiedergegeben werden:

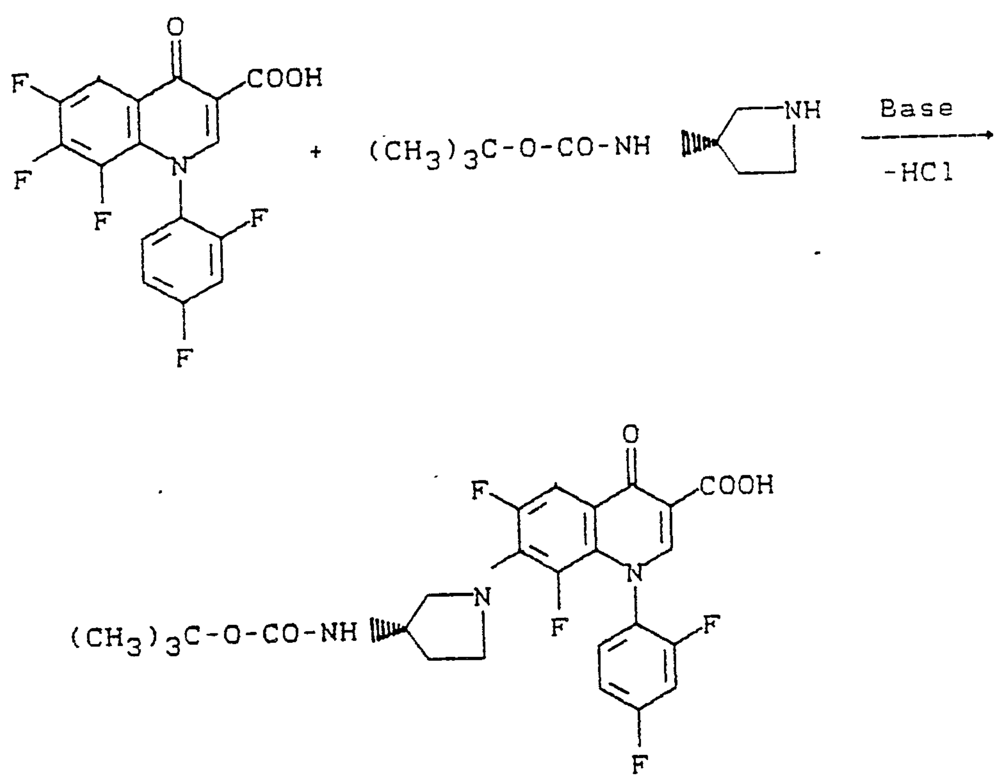

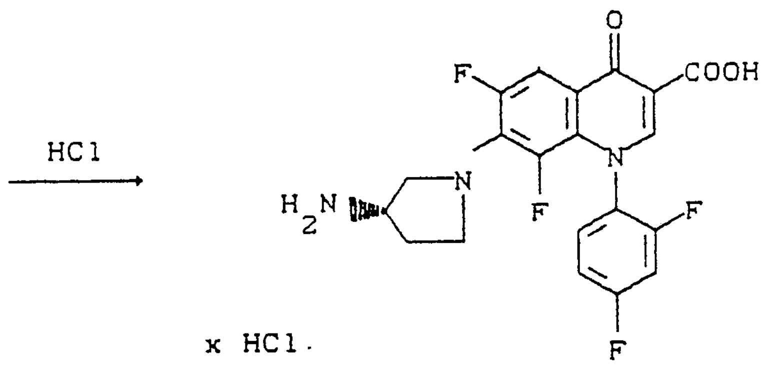


Die als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854).

1-Cyclopropyl-6,7-difluor-1,4-dihydro-4-oxo-3-chinolincarbonsäure (Europäische Patentanmeldung 113 091),

8-Chlor-1-cyclopropyl-6,7-difluor-1,4-dihydro-4-oxo-3-chinolincarbonsäure (Deutsche Patentanmeldung 3 420 743),

1-Cyclopropyl-6,7,8-trifluor-1,4-dihydro-4-oxo-3-chinolincarbonsäure (Deutsche Patentanmeldung 3 318 145),

1-Cyclopropyl-6,7-difluor-1,4-dihydro-8-methyl-4-oxo-3-chinolincarbonsäure,

1-Cyclopropyl-7-chlor-6-fluor-1,4-dihydro-8-nitro-4-oxo-3-chinolincarbonsäure,

6,7-Difluor-1-ethyl-1,4-dihydro-4-oxo-3-chinolincarbonsäure,

7-Chlor-6-fluor-1-ethyl-1,4-dihydro-4-oxo-3-chinolincarbonsäure,

7-Chlor-6-fluor-1,4-dihydro-1-(2-hydroxyethyl)-4-oxo-3-chinolincarbonsäure,

6,7-Difluor-1-(2-fluorethyl)-1,4-dihydro-4-oxo-3-chinolincarbonsäure,

6,7-Difluor-1,4-dihydro-4-oxo-1-phenyl-3-chinolincarbonsäure,

7-Chlor-1-cyclopropyl-6-fluor-1,4-dihydro-4-oxo-1,8-naphthyridin-3-carbonsäure,

1-Cyclopropyl-6,7,8-trifluor-1,4-dihydro-4-oxo-3-chinolincarbonsäureethylester (Deutsche Patentanmeldung 3 318 145),

9,10-Difluor-2,3-dihydro-3-methyl-7-oxo-7H-pyrido[1,2,3-de][1,4]benzoxazin-6-carbonsäure (Europäische Patentanmeldung 47 005),

7-Chlor-6-fluor-1-phenyl-1,4-dihydro-4-oxo-1,8-naphthyridin-3-carbonsäure (Europäische Patentanmeldung 153 580),

7-Chlor-6-fluor-1-(4-fluorphenyl)-1,4-dihydro-4-oxo-1,8-naphthyridin-3-carbonsäure (Europäische Patentanmeldung 153 580),

7-Chlor-6-fluor-1,4-dihydro-8-nitro-4-oxo-1-phenyl-3-chinolincarbonsäure,

7-Chlor-6-fluor-1-(4-fluorphenyl)-1,4-dihydro-8-nitro-4-oxo-3-chinolincarbonsäure,

6,7-Difluor-1-(4-fluorphenyl)-1,4-dihydro-8-methyl-4-oxo-3-chinolincarbonsäure,

6-Chlor-7-fluor-1-(4-fluorphenyl)-1,4-dihydro-4-oxo-3-chinolincarbonsäure (Europäische Patentanmeldung 131 839),

6-Chlor-7-fluor-1-(2,4-difluorphenyl)-1,4-dihydro-4-oxo-3-chinolincarbonsäure (Europäische Patentanmeldung 131 839),

6,7,8-Trifluor-1-(4-fluorphenyl)-1,4-dihydro-4-oxo-3-chinolincarbonsäure (Europäische Patentanmeldung 154 780),

6,7,8-Trifluor-1-(2,4-difluorphenyl)-1,4-dihydro-4-oxo-3-chinolincarbonsäure (Europäische Patentanmeldung 154 780),

6,7,8-Trifluor-1,4-dihydro-4-oxo-1-phenyl-3-chinolincarbonsäure (Europäische Patentanmeldung 154 780),

7-Chlor-1-ethyl-6-fluor-1,4-dihydro-4-oxo-1,8-naphthyridin-3-carbonsäure,

6,7-Difluor-1,4-dihydro-4-oxo-4-vinyl-3-chinolincarbonsäure,

1-Cyclopropyl-5,6,7,8-tetrafluor-1,4-dihydro-4-oxo-3-chinolincarbonsäure,

5-Amino-1-cyclopropyl-6,7,8-trifluor-1,4-dihydro-4-oxo-3-chinolincarbonsäure,

1-Cyclopropyl-6,7-difluor-1,4-dihydro-8-methoxy-4-oxo-3-chinolincarbonsäure.

5,6,7,8-Tetrafluor-1-(2,4-difluorphenyl)-1,4-dihydro-4-oxo-3-chinolincarbonsäure

7-Chlor-6-fluor-1-(2,4-difluorphenyl)-1,4-dihydro-4-oxo-1,8-naphthyridin-3-carbonsäure

Die als Ausgangsverbindungen verwendeten 3-R- beziehungsweise 3-S-Acylamino - und Alkyoxycarbonylaminopyrrolidine (IIIa) beziehungsweise (IIIb) sind neu. Sie lassen sich aus bekannten enantiomerenreinen 3-Hydroxypyrrolidinderivaten (1) [Heterocycles 26, 2247 (1987)] mit Imiden der Formel (2) unter den Bedingungen der Mitsunobu-Reaktion [J. Amer.Chem.Soc.94, 679 (1972)] herstellen. Die erhaltenen Diacylaminopyrrolidinderivate (3) lassen sich zu den freien Aminopyrrolidinen (4) hydrolysieren, worauf die freie Aminfunktion mit einem Acyl- beziehungs weise Alkoxycarbonylrest geschützt werden kann (Weg a). Ebenso lassen sich die erhaltenen Diacylaminopyrrolidinderivate (3) selektiv zu den Acylamino- beziehungsweise Alkoxycarbonylaminopyrrolidinderivaten (5) hydrolysieren (Weg b). Anschließend wird die Schutzgruppe $R^1$ unter Bildung von (IIIa) abgespalten:

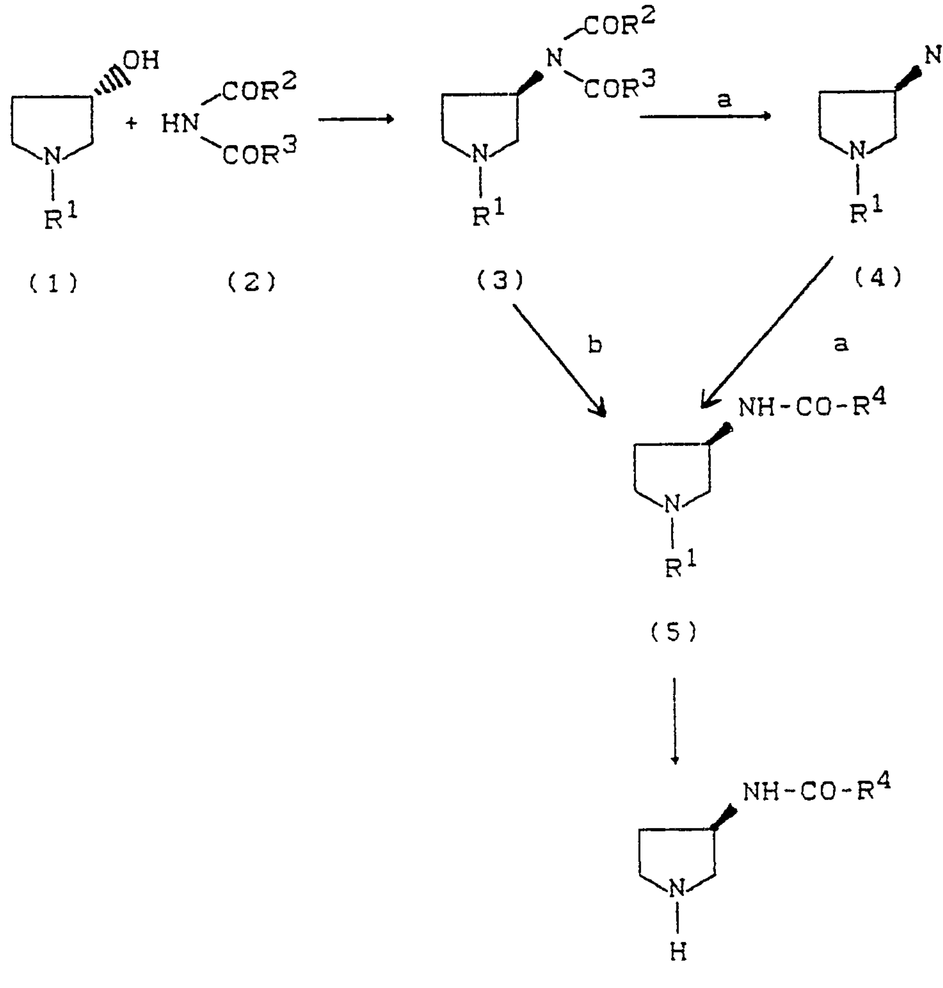


(1) (2) (3) (4)

(5)

(IIIa)

$R^1$ = Benzyl, 1-Phenylethyl, Benzhydryl, Trityl, Acetyl, Trifluoracetyl, $C$-$C_4$-Alkoxycarbonyl, Benzyloxycarbonyl, Benzoyl,
$R^2$, $R^3$, $R^4$ = gleich oder verschieden H, $C_1$-$C_4$-Alkyl, gegebenenfalls durch Halogen, Methyl oder Methoxy, substituiertes Phenyl, $C$-$C_4$-Alkoxy,
wobei zusätzlich $R^2$ und $R^3$ auch gemeinsam eine Brücke der Struktur $-CH_2CH_2-$, $-CH_2CH_2CH_2-$ oder

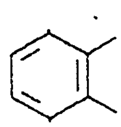

bilden können.

Die Hydrolyse der Diacylaminopyrrolidine (3) kann, je nach Rest $R^1$, sowohl sauer als auch basisch erfolgen. Zur sauren Hydrolyse verwendet man wäßrige Mineralsäuren wie Salzsäure, Bromwasserstoffsäure, Schwefelsäure oder Methansulfonsäure und Trifluoressigsäure; bevorzugt verwendet man Salzsäure, Bromwasserstoffsäure und Schwefelsäure. Zur basischen Hydrolyse werden Alkalimetall- oder Erdalkalimetallhydroxide eingesetzt, bevorzugt sind Lithiumhydroxid, Natriumhydroxid, Kaliumhydroxid, Calciumhydroxid und Bariumhydroxid. Als Lösungsmittel dienen Wasser oder Alkohole, bevorzugt sind Wasser oder Ethanol oder Gemische aus diesen Lösungsmitteln. Die Hydrolyse erfolgt bei Temperaturen zwischen 0° C und 200° C, bevorzugt arbeitet man zwischen 20° C und 140° C. Dabei kann man bei Drucken zwischen 1 bar und 100 bar, bevorzugt zwischen 1 bar und 10 bar arbeiten.

Die weitere Umsetzung zu den Acyl- beziehungsweise Alkoxycarbonyl-aminopyrrolidinderivaten (5) erfolgt mit Acylierungsreagenzien in Gegenwart einer Hilfsbase in einem Lösungsmittel. Als Acylierungsreagenzien dienen Säurehalogenide oder Chlorameisensäureester der Formel $R^4$-CO-X (X= Cl, Br), Anhydride der Formel $(R^4\text{-CO})_2O$ oder gemischte Anhydride der Säuren $R^4$-COOH mit Chlorameisensäureestern oder Sulfonsäuren wie Methansulfonsäure, Benzolsulfonsäure oder 4-Toluolsulfonsäure. Als Hilfsbasen verwendet man Alkalimetallcarbonate oder -hydrogencarbonate, Alkalimetall- oder Erdalkalimetallhydroxide, tertiäre Amine und bicyclische Amidine, bevorzugt verwendet man Natriumcarbonat, Natriumhydrogencarbonat, Kaliumcarbonat, Lithiumhydroxid, Natriumhydroxid, Kaliumhydroxid, Calciumhydroxid, Triethylamin, N,N′-Dimethylanilin, 1,4-Diazabicyclo[2.2.2]octan (Dabco), 1,5-Diazabicyclo[4.3.0]non-5-en, 1,8-Diazabicyclo-[5.4.0]undec-7-en. Als Lösungsmittel kommen in Frage: Wasser, Alkohole, Ether wie Diethylether, Diisopropylether, tert.-Butylmethylether, Tetrahydrofuran oder Dioxan, Kohlenwasserstoffe wie Hexan, Benzol, Toluol, Xylole, und halogenierte Kohlenwasserstoffe wie Methylenchlorid, Chloroform und Chlorbenzol sowie Pyridin, welches gleichzeitig als Hilfsbase dient, und Gemische dieser Lösungsmittel. Bevorzugt sind Wasser, Ethanol, Isopropanol, tert.-Butanol, Diethylether, tert.-Butylmethylether, Tetrahydrofuran, Dioxan, Hexan, Toluol, Methylenchlorid, Chlorbenzol und Pyridin.

Die Umsetzung erfolgt bei Temperaturen zwischen -30° C und 100° C, bevorzugt zwischen -10° C und 50° C.

Die Umsetzung kann bei Drucken zwischen 1 bar und 10 bar, bevorzugt bei 1 bar bis 5 bar durchgeführt werden.

Die direkte Überführung der Diacylaminopyrrolidinderivate (3) in die Acylaminopyrrolidinderivate (5) erfolgt durch basische Hydrolyse, wobei äquimolare Mengen Base verwendet werden. Als Basen verwendet man Alkalimetallhydroxide beziehungsweise -alkoholate. Bevorzugt sind Lithiumhydroxid, Natriumhydroxid, Kaliumhydroxid, Natriummethylat oder Natriumethylat.

Als Lösungsmittel dienen Wasser, $C_1$-$C_4$-Alkohole, Tetrahydrofuran oder Dioxan, bevorzugt arbeitet man in Wasser, Methanol oder Ethanol oder Gemischen dieser Lösungsmittel.

Die Hydrolyse wird bei Temperaturen von 20° C bis 200° C durchgeführt, bevorzugt arbeitet man bei 20° C bis 140° C.

Die Reaktion kann bei Drucken zwischen 1 bar und 100 bar erfolgen, bevorzugt sind 1 bar bis 10 bar.

Die Schutzgruppe $R^1$ kann hydrogenolytisch oder hydrolytisch abgespalten werden. Die Hydrogenolyse wird in Lösungsmitteln wie Wasser, $C_1$-$C_4$-Alkoholen, $C_1$-$C_4$-Carbonsäuren, alkoholischer Salzsäure und Ethern wie Tetrahydrofuran oder Dioxan oder deren Gemischen ausgeführt, bevorzugt sind Methanol, Ethanol, Essigsäure, alkoholische Salzsäure und Tetrahydrofuran. Als Katalysator wird Palladium verwendet, sowohl als Schwamm als auch auf Trägern wie Aktivkohle, Calciumcarbonat oder Bariumsulfat oder als Palladiumhydroxid auf Aktivkohle, bevorzugt ist Palladium auf Aktivkohle. Man arbeitet bei Drucken von 1 bar bis 200 bar und bei Temperaturen zwischen 0° C und 200° C, bevorzugt sind Drucke von 1 bar bis 150 bar und Temperaturen von 20° C bis 140° C.

Die Abspaltung von Acylresten durch Hydrolyse erfolgt unter sauren oder basischen Bedingungen, wobei die Bedingungen so gewählt werden müssen, daß die Schutzgruppe $R^4$-CO- intakt bleibt. Zum Beispiel kann $R^1$ = Trifluoracetyl unter schwach basischen Bedingungen in Gegenwart von $R^4$ = tert.-Butoxy abgespalten werden.

Die enantiomerenreinen 3-Hydroxypyrrolidinderivate der Formel (1) können nach Aktivierung mit einem Carbodiimid unter Walden-Umkehr mit Carbonsäuren verestert, die erhaltenen Ester (6) zum Alkohol mit inverser Konfiguration (7) verseift werden [Angew. Chem. 99, 800 (1987)]. Die Verbindungen (7) lassen sich analog (1) in die S-konfigurierten 3-Acylamino-pyrrolidine (IIIb) überführen:

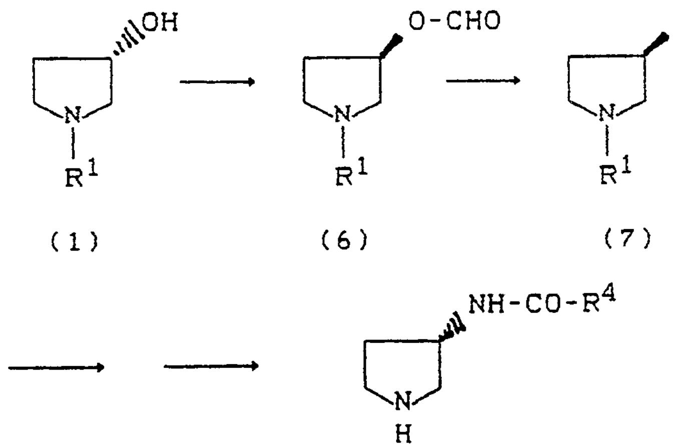

(1) (6) (7)

(IIIb)

Nach diesen Verfahren lassen sich beispielsweise enantiomerenreine S- beziehungsweise R-Formen von folgenden Ausgangsverbindungen (III) herstellen:
3-Formylamino-pyrrolidin
3-Acetylamino-pyrrolidin
3-Propionylamino-pyrrolidin
3-Methoxycarbonylamino-pyrrolidin
3-Ethoxycarbonylamino-pyrrolidin
3-Propoxycarbonylamino-pyrrolidin
3-Butoxycarbonylamino-pyrrolidin
3-tert.-Butoxycarbonylamino-pyrrolidin
3-Isobutoxycarbonylamino-pyrrolidin
3-sek.-Butoxycarbonylamino-pyrrolidin

Die Synthese von S-3-Aminopyrrolidin erfolgt durch Reduktion von S-3-Aminopyrrolidin-2-on (J.Chem.Soc.1951, 104) mit Lithiumaluminiumhydrid.

Die Umsetzung von (II) mit (III) wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200° C, vorzugsweise zwischen 80 und 180° C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 5 Mol, vorzugsweise 1 bis 3 Mol der Verbindung (III) ein.

Aminoschutzgruppen werden nach Beendigung der Umsetzung durch saure oder alkalische Hydrolyse entfernt. Zum Beispiel wird der tert.-Butoxycarbonylrest nach Beendigung der Reaktion durch Behandlung mit einer geeigneten Säure wie Chlorwasserstoffsäure oder Trifluoressigsäure abgespalten (siehe Houben-Weyl, Methoden der organischen Chemie, Band E4, Seite 144 (1983); J.F.W. McOmie, Protective Groups in Organic Chemistry (1973), Seite 43).

Zur Herstellung der erfindungsgemäßen Ester kann die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200° C, vorzugsweise etwa 60° bis 120° C umgesetzt werden. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit

Dimethylformamiddialkylacetal in einem Lösungsmittel wie Dimethylformamid.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können beispielsweise die folgenden Wirkstoffe hergestellt werden:

7-[S-(3-Amino)-1-pyrrolidinyl]-1-cyclopropyl-1,4-dihydro-6-fluor-4-oxo-1,8-naphthyridin-3-carbonsäure,
7-[S-(3-Amino)-1-pyrrolidinyl]-6-fluor-1-(2,4-difluorphenyl)-1,4-dihydro-4-oxo-1,8-naphthyridin-3-carbonsäure,
5-Amino-7-[S-(3-amino)-1-pyrrolidinyl]-1-cyclopropyl-6,8-difluor-1,4-dihydro-4-oxo-3-chinolincarbonsäure,
5-Amino-7-[S-(3-amino)-1-pyrrolidinyl]-1-ethyl-6,8-difluor-1,4-dihydro-4-oxo-3-chinolincarbonsäure,
7-[S-(3-Amino)-1-pyrrolidinyl]-8-chlor-1-cyclopropyl-6-fluor-1,4-dihydro-4-oxo-3-chinolincarbonsäure-ethylester,
7-[S-(3-Amino)-1-pyrrolidinyl]-1-cyclopropyl-6,8-difluor-1,4-dihydro-4-oxo-3-chinolincarbonsäureethylester,
7-[S-(3-Amino)-1-pyrrolidinyl]-1-cyclopropyl-6-fluor-1,4-dihydro-5,8-dimethyl-4-oxo-3-chinolincarbonsäure,
7-[S-(3-Amino)-1-pyrrolidinyl]-1-cyclopropyl-5,6,8-trifluor-1,4-dihydro-4-oxo-3-chinolincarbonsäure,
7-[S-(3-Amino)-1-pyrrolidinyl]-1-cyclopropyl-6-fluor-1,4-dihydro-4-oxo-3-chinolincarbonsäure,
7-[S-(3-Amino)-1-pyrrolidinyl]-1-ethyl-6,8-difluor-1,4-dihydro-4-oxo-3-chinolincarbonsäure,
7-[S-(3-Amino)-1-pyrrolidinyl]-6,8-difluor-1-(2,4-difluorphenyl)-1,4-dihydro-4-oxo-3-chinolincarbonsäure.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephri tis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intesti naltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-

Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lö sungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht

werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37° C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Keimwachstum zu erkennen war.

In der nachfolgenden Tabelle sind die MHK-Werte einiger der erfindungsgemäßen Verbindungen im Vergleich zum entsprechenden Racemat aufgeführt:

MHK-Werte (mg/l) bestimmt durch Agar-Verdünnungstest (Denley Multipoint Inoculator; Iso-Sensitest-Agar)

| Teststamm \ Beispiel | *) | 1 | 2 | **) | 3 | 4 | 5 | 6 |
|---|---|---|---|---|---|---|---|---|
| E. coli Neumann | 0,0019 | ≤ 0,0004 | 0,0019 | 0,0039 | 0,0039 | 0,0078 | ≤ 0,015 | 0,031 |
| 455/7 | 0,125 | 0,125 | 0,25 | 0,25 | 0,25 | 0,5 | 2 | 8 |
| A261 | 0,0019 | 0,0009 | 0,0039 | 0,0039 | 0,0039 | 0,0078 | ≤ 0,015 | 0,031 |
| Klebsiella pneum. 63 | 0,0039 | 0,0039 | 0,0078 | 0,0078 | 0,0078 | 0,015 | 0,031 | 0,062 |
| 8085 | 0,0039 | 0,0039 | 0,0078 | 0,0078 | 0,0078 | 0,015 | 0,031 | 0,062 |
| Proteus mir. 8223 | 0,25 | 0,25 | 0,25 | 0,5 | 0,25 | 0,5 | 4 | 8 |
| 8175 | 0,0078 | 0,0078 | 0,15 | 0,031 | 0,031 | 0,031 | 0,125 | 0,5 |
| Proteus vulg. 1017 | 0,0019 | ≤ 0,0004 | 0,0019 | 0,0039 | 0,0039 | 0,0078 | 0,031 | 0,062 |
| Morg. morg. 932 | 0,0019 | 0,0019 | 0,0039 | 0,0039 | 0,0039 | 0,0078 | 0,031 | 0,062 |
| 11006 | 0,0078 | 0,0039 | 0,0078 | 0,0078 | 0,0078 | 0,015 | 0,062 | 0,125 |
| Providencia stuartei | 0,0039 | 0,0019 | 0,0078 | 0,0078 | 0,0078 | 0,0015 | 0,031 | 0,062 |
| 12052 | 0,125 | 0,125 | 0,25 | 0,25 | 0,25 | 0,5 | 4 | 8 |

(Fortsetzung)

MHK-Werte (mg/l) bestimmt durch Agar-Verdünnungstest (Denley Multipoint Inoculator; Iso-Sensitest-Agar)

| Teststamm | Beispiel *) | 1 | 2 | **) | 3 | 4 | 5 | 6 |
|---|---|---|---|---|---|---|---|---|
| Serratia marcescene 16040 | 0,25 | 0,125 | 0,25 | 0,5 | 0,25 | 1 | 2 | 4 |
| Staph. aurens FK 422 | 0,015 | 0,0078 | 0,015 | 0,031 | 0,015 | 0,031 | 0,031 | 0,062 |
| 1756 | 0,015 | 0,0078 | 0,015 | 0,031 | 0,015 | 0,031 | 0,031 | 0,125 |
| 133 | 0,015 | 0,0078 | 0,015 | 0,031 | 0,015 | 0,031 | 0,031 | 0,125 |
| Enteroc. faecalis 27101 | 0,03 | 0,03 | 0,03 | 0,062 | 0,031 | 0,062 | 0,125 | 1 |
| 9790 | 0,03 | 0,03 | 0,06 | 0,062 | 0,062 | 0,125 | 0,25 | 2 |
| Pseudomonas aerog. Walter | 0,06 | 0,03 | 0,06 | 0,125 | 0,062 | 0,25 | 0,5 | 2 |
| Ellsworth | 0,03 | 0,03 | 0,06 | 0,125 | 0,62 | 0,125 | 0,25 | 1 |

*) = 7-(3-Amino-1-pyrrolidinyl)-8-chlor-1-cyclopropyl-6-fluor-1,4-dihydro-4-oxo-3-chinolincarbonsäure (Racemat)

**)= 7-(3-Amino-1-pyrrolidinyl)-6,8-difluor-1-cyclopropyl-1,4-dihydro-4-oxo-chinolincarbonsäure (Racemat)

Beispiel A

N-(R-3-Pyrrolidinyl)-carbamidsäure-tert.-butylester [R-(3-tert.-Butoxycarbonylamino)-pyrrolidin]

a) R-3-Amino-1-benzylpyrrolidin

Zu 79,7 g (0,45 Mol) S-1-Benzyl-3-hydroxy-pyrrolidin (J.Med. Chem. 29, 2504-2511 (1986)) und 156 g (0,59 Mol) Triphenylphosphin in 650 ml absolutem Tetrahydrofuran tropft man bei 0° C 108 g (0,62 Mol) Azodicarbonsäurediethylester. Dazu gibt man bei 0° C 70,8 g (0,48 Mol) Phthalimid in kleinen Portionen innerhalb von zwei Stunden hinzu. Anschließend rührt man über Nacht bei Raumtemperatur. Man engt ein und löst den Rückstand in 500 ml Essigsäureethylester. Man setzt 1 l Petrolether hinzu und läßt über Nacht auskristallisieren. Die Kristalle, Triphenylphosphinoxid und Hydrazindicarbonsäurediethylester, werden abfiltriert und verworfen, das Filtrat wird eingeengt,
Das auf dieser Stufe anfallende R-1-Benzyl-3-phthalimidopyrrolidin kann durch Chromatographie an Aluminiumoxid in Essigsäureethylester/Petrolether 3:7 gereinigt und aus Essigsäureethylester umkristallisiert werden.
Schmelzpunkt: 118-121° C
$[\alpha]_D^{20}$ = -39,8° (c = 1,013, Methanol)

Die Hauptmenge des erhaltenen Rohprodukts wurde mit 1 l konzentrierter Salzsäure über Nacht unter Rückfluß erhitzt. Man engt ein, nimmt in 200 ml Wasser auf, stellt mit Kaliumcarbonat alkalisch und extrahiert fünfmal mit je 200 ml Chloroform. Man trocknet über Kaliumcarbonat, engt ein und destilliert.
Ausbeute: 51,2 g (65,6 % der Theorie).
Siedepunkt: 76-78° C/0,1 mbar
$[\alpha]_D^{20}$ = -6,84° (c = 1,11, Methanol)

b) N-(R-1-Benzyl-3-pyrrolidinyl)-carbamidsäure-tert.-butylester

Man löst 36 g (0,2 Mol) R-3-Amino-1-benzylpyrrolidin in 135 ml tert.-Butanol und setzt eine Lösung von 8,8 g Natriumhydroxid in 100 ml Wasser hinzu. Dazu tropft man bei Raumtemperatur 47 g (0,21 Mol) Pyrokohlensäuredi-tert.-butylester und rührt anschließend über Nacht. Man extrahiert dreimal mit je 100 ml Chloroform, trocknet über Kaliumcarbonat, engt ein und kristallisiert aus Petrolether um.
Ausbeute: 51,3 g (92,8 % der Theorie)
Schmelzpunkt: 78,8° C
$[\alpha]_D^{20}$ = +18,7° (c = 1,0, Methanol)

c) N-(R-3-Pyrrolidinyl)-carbamidsäure-tert.-butylester

30 g (0,11 Mol) N-(R-1-Benzyl-3-pyrrolidinyl)-carbamidsäure-tert.-butylester werden in 400 ml Ethanol an 5 g Palladium-Aktivkohle (10 % Pd) bei 100° C und 100 bar hydriert. Der Katalysator wird abfiltriert, das Filtrat eingeengt und der Rückstand destilliert.
Ausbeute: 18,5 g (90 % der Theorie)
Siedepunkt: 85° C/0,05 mbar
$[\alpha]_D^{20}$ = +23,4° (c = 1,078, Methanol)

Beispiel B

N-(S-3-Pyrrolidinyl)-carbamidsäure-tert.-butylester [S-(3-tert.-Butoxycarbonylamino)-pyrrolidin]

a) R-1-Benzyl-3-formyloxypyrrolidin

Man mischt 12,5 g (60 mMol) Dicyclohexylcarbodiimid, 5 mg Kupfer-I-chlorid und 8,9 g (50 mMol) S-1-Benzyl-3-hydroxypyrrolidin und rührt drei Tage bei Raumtemperatur. Man setzt 30 ml Toluol zu, erhitzt zum Rückfluß und tropft 2,3 ml (60 mMol) Ameisensäure hinzu. Man erhitzt über Nacht unter Rückfluß, kühlt den Ansatz ab und saugt Dicyclohexylharnstoff ab. Das Filtrat wird mit Natriumhydrogencarbonatlösung gewaschen, über Natriumsulfat getrocknet, eingeengt und der Rückstand destilliert.
Ausbeute: 8,6 g (83 % der Theorie)
Siedepunkt: 93° C/0,2 mbar
$[\alpha]_D^{20}$ = +7,12° (c = 1,067, Methanol)

b) R-1-Benzyl-3-hydroxypyrrolidin

Man erhitzt 63,5 g (0,31 Mol) R-1-Benzyl-3-formyloxypyrrolidin mit 13,6g Natriumhydroxid in 125 ml Wasser drei Stunden unter Rückfluß. Man extrahiert fünfmal mit je 100 ml Methyl-tert.-butylether, trocknet über Kaliumcarbonat, engt ein und destilliert.
Ausbeute: 46,8 g (85 % der Theorie)
Siedepunkt: 85° C/0,01 mbar
$[\alpha]_D^{20}$ = +3,58° (c = 0,923, Methanol)

c) S-3-Amino-1-benzylpyrrolidin

Analog Beispiel Aa wird S-3-Amino-1-benzylpyrrolidin aus R-1-Benzyl-3-hydroxypyrrolidin erhalten.
Siedepunkt: 82° C/0,08 mbar
$[\alpha]_D^{20}$ = +6,06° (c = 1,155, Methanol)

d) N-(S-1-Benzyl-3-pyrrolidinyl)-carbamidsäure-tert.-butylester

Analog Beispiel Ab wird N-(S-1-Benzyl-3-pyrrolidinyl)-carbamidsäure-tert.-butylester aus S-3-Amino-1-benzylpyrrolidin erhalten.
Schmelzpunkt: 77-78° C
$[\alpha]_D^{20}$ = -17,93° (c = 1,227, Methanol)

e) N-(S-3-Pyrrolidinyl)-carbamidsäure-tert.-butylester

Analog Beispiel Ac wird N-(S-3-Pyrrolidinyl)-carbamidsäure-tert.-butylester aus N-(S-1-Benzyl-3-pyrrolidinyl)-carbamidsäure-tert.-butylester erhalten.
Siedepunkt: 90° C/0,1 mbar
$[\alpha]_D^{20}$ = -23,66° (c = 0,9, Methanol)

Beispiel C

S-3-Aminopyrrolidin

Man tropft eine Lösung von 11 g (0,11 Mol) S-3-Aminopyrrolidin-2-on [J.Chem.Soc., 104-108 (1951)] in 10 ml absolutem Dioxan zu 8,4 g (0,22 Mol) Lithiumaluminiumhydrid in 200 ml absolutem Tetrahydrofuran und rührt über Nacht unter Rückfluß. Man zersetzt überschüssiges Lithiumaluminiumhydrid durch vorsichtiges Zutropfen von je 8,5 ml Wasser, 15 %iger wäßriger Kalilauge und 8,5 ml Wasser. Die Salze werden abgesaugt und dreimal mit je 50 ml Tetrahydrofuran ausgekocht. Man trocknet über Kaliumcarbonat, engt ein und destilliert.
Ausbeute: 6,6 g (69,6 % der Theorie),
Siedepunkt: 50° C/10 mbar,
$[\alpha]_D^{20}$ = -12,55°, c = 1,064 (Methanol).

Beispiel 1

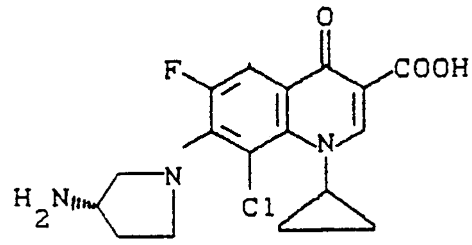

a) Eine Mischung aus 3 g (10 mMol) 8-Chlor-1-cyclopropyl-6,7-difluor-1,4-dihydro-4-oxo-3-chinolincarbonsäure und 2,2 g (20 mMol) 1,4-Diazabicyclo[2.2.2]octan in 25 ml Acetonitril und 12,5 ml DMF wird mit 2 g (10,8 mMol) S-(3-tert.-Butoxycarbonylamino)-pyrrolidin versetzt und 2 Stunden unter Rückfluß erhitzt. Die Mischung wird eingeengt, der Rückstand mit 100 ml Wasser verrührt, der Niederschlag abgesaugt, mit Wasser gewaschen und bei 70 °C im Vakuum getrocknet. Das isolierte Rohprodukt (4,6 g) wird aus Dimethylformamid umkristallisiert, mit Ethanol gewaschen und bei 100 °C im Vakuum getrocknet.
Ausbeute: 3,2 g (68,7 % der Theorie) 7-[S-(3-tert.-Butoxycarbonylamino)-1-pyrrolidinyl]-8-chlor-1-cyclopropyl-6-fluor-1,4-dihydro-4-oxo-3-chinolincarbonsäure, Schmelzpunkt: 198-200 °C (unter Zersetzung), $[\alpha]_D^{20}$ = -53,5° (c = 1, $CHCl_3$)

b) 2,7 g (5,8 mMol) des Produkt aus Stufe A werden bei Raumtemperatur in 6 ml Trifluoressigsäure eingetragen. Nach 45 Minuten wird die Lösung eingeengt, der zähe Rückstand mit 25 ml Methylenchlorid verrührt und nochmals im Vakuum eingeengt. Anschließend wird der Rückstand mit 30 ml Diethylether verknetet bis die ganze Masse durchkristallisiert ist. Der Niederschlag wird abgesaugt, mit Diethylether gewaschen und getrocknet.
Ausbeute: 2,6 g (93,5 % der Theorie) 7-[S-(3-Amino)-1-pyrrolidinyl]-8-chlor-1-cyclopropyl-6-fluor-1,4-dihydro-4-oxo-3-chinolincarbonsäure-Trifluoracetat, Schmelzpunkt: 237-238 °C (unter Zersetzung), $[\alpha]_D^{20}$ = +27,6° (c = 1,095, DMF).

c) 2,5 g (5,2 mMol) des Produktes aus Stufe B werden in 120 ml Wasser bei 40 °C gelöst, die Lösung über eine Membran (regenerierte Zellulose, Porengröße 0,45 µm) filtriert und das Filtrat mit 8,5 %iger wäßriger Ammoniaklösung neutral gestellt. Das ausfallende Betain wird abgesaugt, gut mit Wasser gewaschen und bei 80 °C im Vakuum getrocknet.
Ausbeute: 1,9 g (100 % der Theorie) 7-[S-(3-Amino)-1-pyrrolidinyl]-8-chlor-1-cyclopropyl-6-fluor-1,4-dihydro-4-oxo-3-chinolincarbonsäure-Trifluoracetat, Schmelzpunkt: 252-253 °C (unter Zersetzung), $[\alpha]_D^{20}$ = +1,59° (c = 0,565, 1n HCl).

d) 1,6 g (4,4 mMol) des Produkts aus Stufe C (Betain) werden in 8 ml Wasser suspendiert und mit 4,4 ml 1 n-Salzsäure versetzt. Das gebildete Hydrochlorid wird unter leichtem Erwärmen in Lösung gebracht, die Lösung durch Filtration über eine Membran (regenerierte Zellulose, Porengröße 0,45 µm) geklärt und das Filtrat gefriergetrocknet. Das erhaltende Lyophilisat wird mit 13 ml Ethanol verrührt, wobei es rekristallisiert. Das Hydrochlorid wird abgesaugt, mit Ethanol gewaschen und getrocknet.
Ausbeute: 1,4 g (76,6 % der Theorie) 7-[S-(3-Amino)-1-pyrrolidinyl]-8-chlor-1-cyclopropyl-6-fluor-1,4-dihydro-4-oxo-3-chinolincarbonsäure-Hydrochlorid, Schmelzpunkt: 267-268 °C (unter Zersetzung), $[\alpha]_D^{20}$ = +0,86° (c = 0,465, 1n HCl).

Beispiel 2

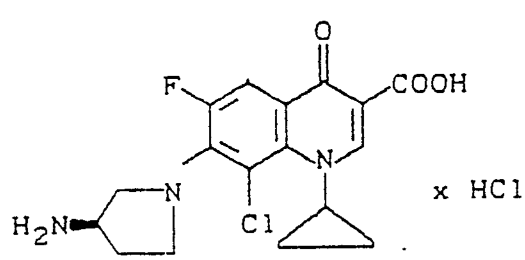

Analog Beispiel 1 wird die Umsetzung mit R-(3-tert.-Butoxy-carbonylamino)-pyrrolidin durchgeführt.

a) 7-[R-(3-tert.-Butoxycarbonylamino)-1-pyrrolidinyl]-8-chlor-1-cyclopropyl-6-fluor-1,4-dihydro-4-oxo-3-chinolincarbonsäure, Schmelzpunkt: 198-200 °C (unter Zersetzung), $[\alpha]_D^{20}$ = +52,0° (c = 1,025, $CHCl_3$).

b) 7-[R-(3-Amino)-1-pyrrolidinyl]-8-chlor-1-cyclopropyl-6-fluor-1,4-dihydro-4-oxo-3-chinolincarbonsäure -Trifluoracetat, Schmelzpunkt: 235-237 °C (unter Zersetzung), $[\alpha]_D^{20}$ = -26,6° (c = 1,075, DMF).

c) 7-[R-(3-Amino)-1-pyrrolidinyl]-8-chlor-1-cyclopropyl-6-fluor-1,4-dihydro-4-oxo-3-chinolincarbonsäure, Schmelzpunkt: 257-258 °C (unter Zersetzung), $[\alpha]_D^{20}$ = -1,3° (c = 0,455, 1n HCl).

d) 7-[R-(3-Amino)-1-pyrrolidinyl]-8-chlor-1-cyclopropyl-6-fluor-1,4-dihydro-4-oxo-3-chinolincarbonsäure -Hydrochlorid, Schmelzpunkt: 270-271 °C (unter Zersetzung), $[\alpha]_D^{20}$ = -1,29° (c = 0,465, 1n HCl).

Beispiel 3

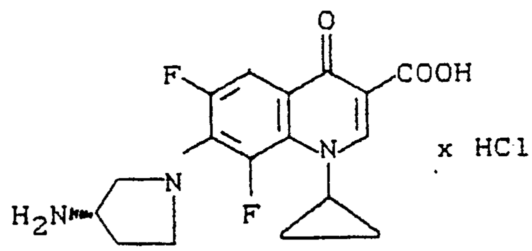

a) Analog Beispiel 1a wird mit 1-Cyclopropyl-6,7,8-trifluor-1,4-dihydro-4-oxo-3-chinolincarbonsäure zu 7-[S-(3-tert.-Butoxy-carbonylamino)-1-pyrrolidinyl]-1-cyclopropyl-6,8-difluor-1,4-dihydro-4-oxo-3-chinolincarbonsäure umgesetzt; Schmelzpunkt: 232-235 °C (unter Zersetzung), $[\alpha]_D^{20}$ = -47,3° (c = 0,99, $CHCl_3$).

b) 4,1 g (9,1 mMol) des Produkts aus Beispiel 3a werden in einer Mischung aus 47 ml Methanol und 47 ml konzentrierter Salzsäure 30 Minuten bei Raumtemperatur gerührt. Die Lösung wird eingeengt, der Rückstand mit 60 ml Ethanol aufgekocht, gekühlt, der Niederschlag abgesaugt, mit Ethanol gewaschen und bei 100° im Vakuum getrocknet.
Ausbeute: 2,93 g (83 % der Theorie) 7-[S-(3-Amino)-1-pyrrolidinyl]-1-cyclopropyl-6,8-difluor-1,4-dihydro-4-oxo-3-chinolincarbonsäure-Hydrochlorid, Schmelzpunkt: 310-312 °C (unter Zersetzung), $[\alpha]_D^{20}$ = -54,8° (c = 0,349, $H_2O$).

c) Eine Mischung von 2,83 g (10 mMol) 1-Cyclopropyl-6,7,8-trifluor-1,4-dihydro-3-chinolincarbonsäure und 2,2 g (20 mMol) 1,4-Diazabicyclo[2.2.2]octan in 20 ml Acetonitril und 10 ml Dimethylformamid wird mit 1,3 g (15 mMol) S-3-Amino-pyrrolidin versetzt und 2 Stunden unter Rückfluß erhitzt. Die Suspension wird eingeengt, der Rückstand mit Wasser verrührt, der Niederschlag abgesaugt, mit Wasser gewaschen und getrocknet.
Man erhält 3,1 g Rohprodukt, das aus Dimethylformamid umkristallisiert wird.
Ausbeute: 2,8 g (80 % der Theorie) 7-[S-(3-Amino)-1-pyrrolidinyl]-1-cyclopropyl-6,8-difluor-1,4-dihydro-4-oxo-3-chinolincarbonsäure, Schmelzpunkt: 282-285 °C (unter Zersetzung).
700 g (2 mMol) des Betains werden in 7 ml halbkonzentrierter Salzsäure in der Wärme gelöst, die Lösung filtriert und in dem Filtrat das Hydrochlorid durch Zugabe von Ethanol ausgefällt.
Ausbeute: 630 mg (82 % der Theorie) 7-[S-(3-Amino)-1-pyrrolidinyl]-1-cyclopropyl-6,8 difluor-1,4-dihydro-4-oxo-3-chinolincarbonsäure-Hydrochlorid, Schmelzpunkt: 310-312 °C (unter Zersetzung), $[\alpha]_D^{20}$ = -56,7° (c = 0,24, $H_2O$).

Beispiel 4

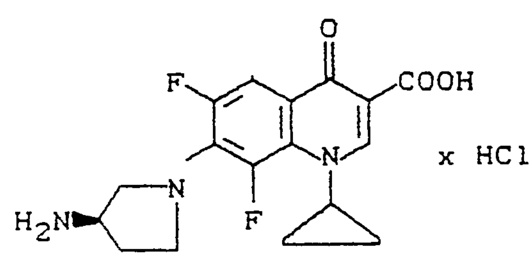

Analog Beispiel 3 wird die Umsetzung mit R-(3-tert.-Butoxycarbonylamino)-pyrrolidin durchgeführt:

a) 7-[R-(3-tert.-Butoxycarbonylamino)-1-pyrrolidinyl]-1-cyclopropyl-6,8-difluor-1,4-dihydro-4-oxo-3-chinolincarbonsäure, Schmelzpunkt: 235-236 °C (unter Zersetzung), $[\alpha]_D^{20}$ = +47° (c = 1, $CHCl_3$).

b) 7-[R-(3-Amino)-1-pyrrolidinyl]-1-cyclopropyl-6,8-difluor-1,4-dihydro-4-oxo-3-chinolincarbonsäure-Hydrochlorid, Schmelzpunkt: 305-310 °C (unter Zersetzung), $[\alpha]_D^{20}$ = +50° (c = 0,595, $H_2O$).

Beispiel 5

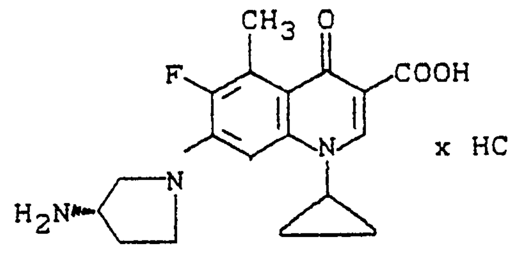

x HCl

a) Eine Mischung von 0,56 g (2 mMol) 1-Cyclopropyl-6,7-difluor-1,4-dihydro-5-methyl-4-oxo-3-chinolincarbonsäure und 0,67 g (6 mMol) 1,4-Diazabicyclo[2.2.2]octan in 7 ml Dimethylsulfoxid wird mit 0,39 g (2,1 mMol) S-(3-tert.-Butoxycarbonylamino)pyrrolidin versetzt und 2 Stunden auf 140 °C erhitzt. Die Mischung wird eingeengt, der Rückstand mit Wasser verrührt, der Niederschlag abgesaugt, mit Wasser gewaschen und bei 70° im Vakuum getrocknet.

Ausbeute: 0,80 g (91 % der Theorie) 7-[S-(3-tert.-Butoxycarbonylamino)-1-pyrrolidinyl]-1-cyclopropyl-6-fluor-1,4-dihydro-5-methyl-4-oxo-3-chinolincarbonsäure, Schmelzpunkt: 232-234 °C (unter Zersetzung).

b) 0,6 g (1,35 mMol) des Produktes aus Stufe a werden in einem Gemisch aus 6 ml Methanol und 6 ml konzentrierter Salzsäure 1 Stunde bei 20 °C gerührt. Danach wird im Vakuum eingeengt. Der Rückstand wird mit Ethanol verrührt, der Niederschlag wird abgesaugt, mit Ethanol gewaschen und im Vakuum getrocknet.

Ausbeute: 0,44 g (86 % der Theorie) 7-[S-(3-Amino)-1-pyrrolidinyl]-1-cyclopropyl-6-fluor-1,4-dihydro-5-methyl-4-oxo-3-chinolincarbonsäure-Hydrochlorid. Schmelzpunkt: 312-314 °C (unter Zersetzung). $[\alpha]_D^{20}$ = +13,1° (c = 0,435; 1n NaOH).

Beispiel 6

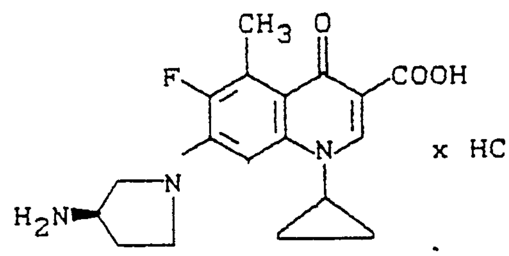

x HCl

Analog Beispiel 5 wird die Umsetzung mit R-(3-tert.-Butoxycarbonylamino)-pyrrolidin durchgeführt:

a) 7-[R-(3-tert.-Butoxycarbonylamino)-1-pyrrolidinyl]-1-cyclopropyl-6-fluor-1,4-dihydro-5-methyl-4-oxo-3-chinolincarbonsäure, Schmelzpunkt: 232-234 °C (unter Zersetzung).

b) 7-[R-(3-Amino)-1-pyrrolidinyl]-1-cyclopropyl-6-fluor-1,4-dihydro-5-methyl-4-oxo-3-chinolincarbonsäure-Hydrochlorid, Schmelzpunkt: 312-314 °C (unter Zersetzung), $[\alpha]_D^{20}$ = -14,8° (c = 0,445, 1n -NaOH).

## Ansprüche

1. Enantiomerenreine Verbindungen der Formel (I)

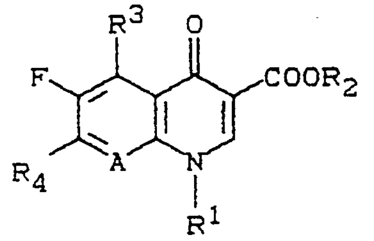

(I) ,

in welcher

$R^1$ für verzweigtes oder geradkettiges Alkyl mit 1 bis 3 Kohlenstoffatomen, Cyclopropyl, Vinyl, 2-Hydroxyethyl, 2-Fluorethyl, gegebenenfalls durch 1 oder 2 Fluoratome substituiertes Phenyl,

$R^2$ für Wasserstoff oder Alkyl mit 1 bis 4 Kohlenstoffatomen,

$R^3$ für Wasserstoff, Fluor, Chlor, Amino, Alkyl mit 1 bis 4 Kohlenstoffatomen,

$R^4$ für einen Rest der Struktur

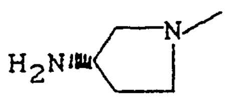 oder 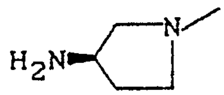 und

A für N oder C-$R^5$ steht, worin $R^5$ für Wasserstoff, Halogen, Methyl, Nitro, Hydroxy oder Methoxy steht oder auch gemeinsam mit $R^1$ eine Brücke der Struktur

$-O-CH_2-CH-CH_3$

bilden kann,

und deren pharmazeutisch verwendbaren Hydrate und Säureadditionssalze sowie die Alkali-, Erdalkali-, Silber-und Guanidiniumsalze der zugrundeliegenden Carbonsäuren, ausgenommen 7-[R- und S-(3-Amino)-1-pyrrolidinyl]-6-fluor-1-(2,4-difluor-phenyl)-1,4-dihydro-4-oxo-1,8-naphthyridin-3-carbonsäure.

2. Verbindungen der Formel (I)

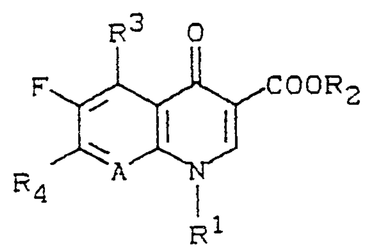

(I) ,

in welcher

$R^1$ für Methyl, Ethyl, Cyclopropyl, 2-Fluorethyl, 4-Fluorphenyl oder 2,4-Difluorphenyl,

$R^2$ für Wasserstoff, Methyl oder Ethyl,

$R^3$ für Wasserstoff, Fluor, Amino oder Methyl,

$R^4$ für einen Rest der Struktur

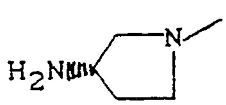 oder 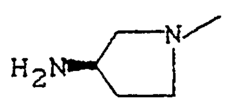 und

A für N oder C-$R^5$ steht, worin $R^5$ für Wasserstoff, Fluor, Chlor, Methyl oder Methoxy steht oder auch gemeinsam mit $R^1$ eine Brücke der Struktur

$-O-CH_2-CH-CH_3$

bilden kann,

und deren pharmazeutisch verwendbaren Hydrate und Säureadditionssalze sowie die Alkali-, Erdalkali-, Silber-und Guanidiniumsalze der zugrundeliegenden Carbonsäuren, ausgenommen 7-[R- und S-(3-Amino)-1-pyrrolidinyl)-6-fluor-1-(2,4-difluor-phenyl)-1,4-dihydro-4-oxo-1,8-naphthyridin-3-carbonsäure.

3. Verbindungen der Formel (I)

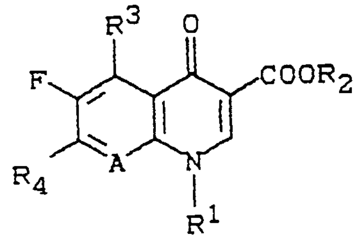

(I),

in welcher

$R^1$ für Methyl, Ethyl, Cyclopropyl, 2-Fluorethyl, 4-Fluorphenyl oder 2,4-Difluorphenyl,

$R^2$ für Wasserstoff, Methyl oder Ethyl,

$R^3$ für Wasserstoff, Fluor, Amino oder Methyl

$R^4$ für einen Rest der Struktur

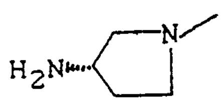

und

A für N oder C-$R^5$ steht, worin $R^5$ für Wasserstoff, Fluor, Chlor, Methyl oder Methoxy steht oder auch gemeinsam mit $R^1$ eine Brücke der Struktur

$-O-CH_2-\ CH-CH_3$

bilden kann,

und deren pharmazeutisch verwendbaren Hydrate und Säureadditionssalze sowie die Alkali-, Erdalkali-, Silber-und Guanidiniumsalze der zugrundeliegenden Carbonsäuren, ausgenommen 7-[S-(3-Amino)-1-pyrrolidinyl]-6-fluor-1-(2,4-difluor-phenyl)-1,4-dihydro-4-oxo-1,8-naphthyridin-3-carbonsäure.

4. Verfahren zur Herstellung von Verbindungen der formel (I) gemäß Anspruch 1, dadurch gekennzeichnet, daß man Verbindungen der Formel (II)

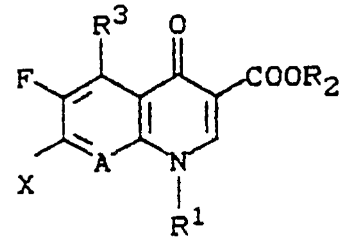

(II),

in welcher $R^1$, $R^2$, $R^3$ und A die in Anspruch 1 angegebene Bedeutung haben und

X für Halogen, insbesondere Fluor oder Chlor steht,

mit Verbindungen der Formel (III) in der R- oder in der S-konfigurierten Form

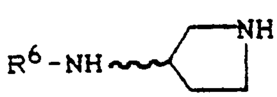

(III),

in welcher

$R^6$ für Wasserstoff, Acyl mit 1 bis 3 Kohlenstoffatomen oder Alkoxycarbonyl mit 1 bis 4 Kohlenstoffatomen im Alkoxyteil, insbesondere tert.-Butoxycarbonyl, steht,

gegebenenfalls in Gegenwart von Säurefängern umsetzt und gegebenenfalls vorhandene Schutzgruppen abspaltet.

5. Verbindungen der Formel (I)

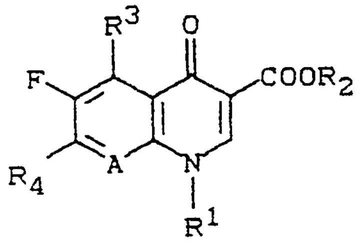

(I) ,

in welcher
$R^1$ für verzweigtes oder geradkettiges Alkyl mit 1 bis 3 Kohlenstoffatomen, Cyclopropyl, Vinyl, 2-Hydroxyethyl, 2-Fluorethyl, gegebenenfalls durch 1 oder 2 Fluoratome substituiertes Phenyl,
$R^2$ für Wasserstoff oder Alkyl mit 1 bis 4 Kohlenstoffatomen,
$R^3$ für Wasserstoff, Fluor, Chlor, Amino, Alkyl mit 1 bis 4 Kohlenstoffatomen,
$R^4$ für einen Rest der Struktur

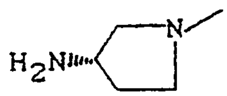 oder 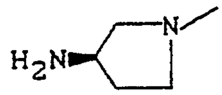 und

A für N oder C-$R^5$ steht, worin $R^5$ für Wasserstoff, Halogen, Methyl, Nitro, Hydroxy oder Methoxy steht oder auch gemeinsam mit $R^1$ eine Brücke der Struktur
$-O-CH_2-\ CH-CH_3$
bilden kann,
und deren pharmazeutisch verwendbaren Hydrate und Säureadditionssalze sowie die Alkali-, Erdalkali-, Silber-und Guanidiniumsalze der zugrundeliegenden Carbonsäuren zur Anwendung in einem Verfahren zur therapeutischen Behandlung des menschlischen oder tierischen Körpers.

6. Verwendung von Verbindungen der Formel (I) gemäß Anspruch 1 bei der Herstellung von Arzneimitteln.

7. Arzneimittel, enthaltend Verbindungen der Formel (I) gemäß Anspruch 1.

8. Verbindungen aus der Gruppe bestehend aus
7-[S-(3-Amino)-1-pyrrolidinyl]-1-cyclopropyl-1,4-dihydro-6-fluor-4-oxo-1,8-naphthyridin-3-carbonsäure,
5-Amino-7-[S-(3-amino)-1-pyrrolidinyl]-1-cyclopropyl-6,8-difluor-1,4-dihydro-4-oxo-3-chinolincarbonsäure,
5-Amino-7-[S-(3-amino)-1-pyrrolidinyl]-1-ethyl-6,8-difluor-1,4-dihydro-4-oxo-3-chinolincarbonsäure,
7-[S-(3-Amino)-1-pyrrolidinyl]-8-chlor-1-cyclopropyl-6-fluor-1,4-dihydro-4-oxo-3-chinolincarbonsäure-ethylester,
7-[S-(3-Amino)-1-pyrrolidinyl]-1-cyclopropyl-6,8-difluor-1,4-dihydro-4-oxo-3-chinolincarbonsäureethylester,
7-[S-(3-Amino)-1-pyrrolidinyl]-1-cyclopropyl-6-fluor-1,4-dihydro-5,8-dimethyl-4-oxo-3-chinolincarbonsäure,
7-[S-(3-Amino)-1-pyrrolidinyl]-1-cyclopropyl-5,6,8-trifluor-1,4-dihydro-4-oxo-3-chinolincarbonsäure,
7-[S-(3-Amino)-1-pyrrolidinyl]-8-chlor-1-cyclopropyl-6-fluor-1,4-dihydro-4-oxo-3-chinolincarbonsäure,
7-[S-(3-Amino)-1-pyrrolidinyl]-8-chlor-1-cyclopropyl-6-fluor-1,4-dihydro-4-oxo-3-chinolincarbonsäure-Trifluoracetat,
7-[S-(3-Amino)-1-pyrrolidinyl]-8-chlor-1-cyclopropyl-6-fluor-1,4-dihydro-4-oxo-3-chinolincarbonsäure,
7-[S-(3-Amino)-1-pyrrolidinyl]-8-chlor-1-cyclopropyl-6-fluor-1,4-dihydro-4-oxo-3-chinolincarbonsäure-Hydrochlorid,
7-[S-(3-tert.-Butoxy-carbonylamino)-1-pyrrolidinyl] -1-cyclopropyl-6,8-difluor-1,4,-dihydro-4-oxo-3-chinolincarbonsäure,
7-[S-(3-Amino)-1-pyrrolidinyl]-1-cyclopropyl-6,8-difluor-1,4-dihydro-4-oxo-3-chinolincarbonsäure-Hydrochlorid,
7-[S-(3-Amino)-1-pyrrolidinyl]-1-cyclopropyl-6,8-difluor-1,4-dihydro-4-oxo-3-chinolincarbonsäure,
7-[S-(3-Amino)-1-pyrrolidinyl]-1-cyclopropyl-6,8-difluor-1,4-dihydro-4-oxo-3-chinolincarbonsäure-Hydrochlorid,
7-[R-(3-tert.-Butoxycarbonylamino)-1-pyrrolidinyl]-1-cyclopropyl-6,8-difluor-1,4-dihydro-4-oxo-3-chinolincarbonsäure,
7-[R-(3-Amino)-1-pyrrolidinyl] -1-cyclopropyl-6,8-difluor-1,4-dihydro-4-oxo-3-chinolincarbonsäure-Hydrochlorid,
7-[R-(3-tert.-Butoxycarbonylamino)-1-pyrrolidinyl]-1-cyclopropyl-6-fluor-1,4-dihydro-5-methyl-4-oxo-3-chinolincarbonsäure,
7-[S-(3-Amino)-1-pyrrolidinyl] -1-cyclopropyl-6-fluor-1,4-dihydro-5-methyl-4-oxo-3-chinolincarbonsäure-Hydrochlorid.
7-[R-(3-tert.-Butoxycarbonylamino)-1-pyrrolidinyl]-1-cyclopropyl-6-fluor-1,4-dihydro-5-methyl-4-oxo-3-chinolincarbonsäure,
7-[R-(3-Amino)-1-pyrrolidinyl]-1-cyclopropyl-6-fluor-1,4-dihydro-5-methyl-4-oxo-3-chinolincarbonsäure-Hydrochlorid,

9. S- und R-Formen der Verbindungen:
3-Formylamino-pyrrolidin
3-Propionylamino-pyrrolidin
3-Methoxycarbonylamino-pyrrolidin
3-Ethoxycarbonylamino-pyrrolidin
3-Propoxycarbonylamino-pyrrolidin
3-Butoxycarbonylamino-pyrrolidin
3-tert.-Butoxycarbonylamino-pyrrolidin
3-Isobutoxycarbonylamino-pyrrolidin
3-sek.-Butoxycarbonylamino-pyrrolidin

10. Verbindungen aus der Gruppe bestehend aus
N-(R-3-Pyrrolidinyl)-carbamidsäure-tert.-butylester
N-(R-1-Benzyl-3-pyrrolidinyl)-carbamidsäure-tert.-butylester
N-(S-3-Pyrrolidinyl)-carbamidsäure-tert.-butylester
N-(S-1-Benzyl-3-pyrrolidinyl)-carbamidsäure-tert.-butylester

11. Verbindungen aus der Gruppe bestehend aus
R-3-Amino-1-benzylpyrrolidin, R-1-Benzyl-3-formyloxypyrrolidin, R-1-Benzyl-3-hydroxy-pyrrolidin und S-3-Amino-1-benzyl-pyrrolidin.